# EUROPEAN PATENT SPECIFICATION

(11) **EP 4 401 821 B1**
(45) Date of publication and mention of the grant of the patent: **20.08.2025**
(21) Application number: 22768759.7
(22) Date of filing: 23.08.2022
(51) Int. Cl.: A61N 1/04, A61B 5/24, A61B 5/00

(54) **AN ELECTRODE PAD FOR ELECTRO-STIMULATION AND METHOD OF MANUFACTURING SAME**
ELEKTRODENPAD ZUR ELEKTROSTIMULATION UND VERFAHREN ZUR HERSTELLUNG DAVON
PLOT D'ÉLECTRODE POUR ÉLECTRO-STIMULATION ET SON PROCÉDÉ DE FABRICATION

(30) Priority: 16.09.2021 NO 20211112
(43) Date of publication of application: 24.07.2024
(73) Proprietor: Luzmon Medical AS, 0258 Oslo (NO)
(72) Inventor: BROWN, David, 0198 Oslo (NO)
(74) Representative: Acapo Onsagers AS
(86) International application number: PCT/EP2022/073441
(87) International publication number: WO 2023/041293

(56) References cited:
- US-A1- 2004 162 602
- US-A1- 2019 134 393
- US-A1- 2019 217 078
- US-A1- 2020 305 746
- US-A1- 2020 397 372

## Description

### Technical field

The present invention relates to electrodes for electro-therapy, in particular an electrode pad for electro-stimulation and a method of manufacturing the electrode pad.

### Background

TENS (Transcutaneous Electrical Nerve Stimulation) and (NMES Neuromuscular Electrical Stimulation) devices comprises a pulse generator connected to a plurality of electrode pads attached to the skin of a patient. It is common to use single-patient and limited re-use electrode pads as the anode and cathode on the patient's skin. The single-patient and limited re-use electrodes typically consist of an electrically conductive sticky hydrogel layer, a similarly sized conductive sheet (typically carbon or graphite) and an external cable connection which is embedded between the sticky hydrogel and the carbon / graphite sheet, the core of the cable being either carbon fibers or copper wires. On the exposed, non-patient, side of the electrode pad is a woven fabric which serves as a substrate / reinforcement and also an insulator to protect the electrical stimulation pulses from unwanted contact.

The single-patient and limited re-use sticky electrodes can be located by the clinician in suitably appropriate areas of limbs or torso in order to stimulate nerves which in turn then either provide pain relief (TENS) or activate muscles (NMES) to provide strengthening of damaged or perhaps inactive muscles. After each treatment of typically 20-40 minutes the sticky electrodes are unpeeled from the patient's skin and normally are disposed of. After each treatment the sticky electrodes are unpeeled from the patient's skin and normally are disposed of. In some cases the clinician may choose to re-use the pads for further treatments on the same patient, however the sticky electrode gel will dry out over time and will therefore have a decline in there electrical conductivity as well as the ability to stick to skin. Re-use might be limited in practical terms to five usages after which the electrode is disposed of and new fresh electrodes used.

Due to the design of the prior art sandwich construction, the distribution of the stimulation pulse declines in intensity as the size of the electrode increases. Some manufacturers of larger electrodes (50x100mm and larger) use double cable connections, equivalent to two co-joined 50x50mm electrodes. Other manufacturers use a longer exposed section of carbon or copper wire in contact with the carbon / graphite sheet. Commercially available pulse generation devices which feel comfortable for the patient when used with 50x50mm single-patient limited re-use electrodes might feel less comfortable when using larger single-patient electrodes as described, the discomfort being apparent in the stimulated limb as a sharp and prickly feeling, often located in one end of the larger electrode.

Further, treatment of different limbs or body parts, on differently sized patients, requires an ability to stimulate differently sized nerves and thus activate differently sized muscles. Prior art electrodes of the above described construction become less effective as they increase in size.

Thus, a need exist for a design and construction of electrode which is multi-patient and multi-reusable, and can be suitably cleaned and disinfected such that it can be used in a hospital or treatment clinic environment across different patients. Preferably, such an electrode would also be able to be scaled in size and improve patient comfort due to more even current distribution without hotspots or edge effects.

Exemplary prior art electrode pads are disclosed in US2020397372, US2004162602, US2019217078, US2019134393 and US2020305746.

### Summary

In view of the above, an object of the present disclosure is to overcome or at least mitigate drawbacks.

The present invention is set forth and characterized in the independent claims, while the dependent claims describe other characteristics of the invention.

In a first aspect the invention relates to an electrode pad for electro-stimulation, comprising:
- a first non-conductive flexible substrate comprising at least one conductive track disposed on a first side of the first non-conductive flexible substrate,
- a primer disposed on the first side of the first non-conductive flexible substrate in areas not covered by the at least one conductive track,
- a layer of conductive silicone disposed over the at least one conductive track and the primer, wherein the primer is bonding the first non-conductive flexible substrate to the conductive silicone providing mechanical contact between the at least one conductive track and a first side of the layer of conductive silicone.

In one embodiment, the electrode pad may further comprising an adhesive disposed on a second side of the first non-conductive flexible substrate, and a second non-conductive flexible substrate comprising at least one conductive track disposed on a first side of the second non-conductive flexible substrate, and where a second side of the second non-conductive flexible substrate, opposite the first side of the second non-conductive flexible substrate, is arranged on the adhesive.

In one embodiment, the electrode pad may further comprise a non-conductive silicone layer covering all sides of the electrode pad other than the second side of the layer of conductive silicone.

In one embodiment, the primer may be a non-conductive primer.

In one embodiment, the layer of layer of conductive silicone may be a compression molded layer of conductive silicone.

In one embodiment, the non-conductive flexible substrate may be a polymer printed circuit board.

In one embodiment, the polymer printed circuit board may comprise at least one microcontroller in operational connection with circuitry adapted for at least one of measure and regulate heat, measure movement, and receive and display a pad-ID.

In one embodiment, the at least one conductive track on the first side of the second non-conductive flexible substrate may be a heating element. The heating element may comprise at least one conductive track arranged in a meandering pattern.

In one embodiment, the electrode pad may comprise at least two conductive tracks, wherein each of the conductive tracks is a separate heating element. The heating elements may comprise at least one conductive track arranged in a meandering pattern.

In one embodiment, the adhesive may be an adhesive film.

In a second aspect the invention provides a method of manufacturing an electrode pad for electro-stimulation, the method comprising:
- providing a first non-conductive flexible substrate comprising at least one conductive track disposed on a first side of the first non-conductive flexible substrate,
- covering the at least one conductive track with a mask,
- disposing a primer on the first side of the first non-conductive flexible substrate,
- removing the mask,
- disposing a layer of conductive silicone over the at least a first conductive track and the primer, such that the primer is bonding the first non-conductive flexible substrate to the conductive silicone providing mechanical contact between the at least one conductive track and a first side of the layer of conductive silicone.

In one embodiment, the method may further comprising disposing an adhesive on a second side of the first non-conductive flexible substrate, providing a second non-conductive flexible substrate comprising at least one conductive track disposed on a first side of the second non-conductive flexible substrate, and arranging a second side of the second non-conductive flexible substrate, opposite the first side of the second non-conductive flexible substrate, on the adhesive.

In one embodiment, the method may further comprise covering all sides of the electrode pad other than the second side of the layer of conductive silicone with a non-conductive silicone layer.

In one embodiment, the step of disposing a layer of conductive silicone may further comprising placing the first non-conductive flexible substrate in a molding tool, disposing conductive silicone over the at least a first conductive track and the primer in the molding tool, and applying pressure and heat to the molding tool, such that the layer of conductive silicone is compression molded to the first non-conductive flexible substrate.

### Brief description of the drawings

Figure 1 illustrates a cross-section of an exemplary electrode pad according to an embodiment of the present invention.
Figure 2 illustrates a cross-section of an exemplary electrode pad according to an embodiment of the present invention.
Figure 3 illustrates top views of exemplary electrode pads according to embodiments of the present invention.
Figure 4 illustrates top views of exemplary electrode pads according to embodiments of the present invention.
Figure 5 illustrates a top views of an exemplary electrode pad according to embodiments of the present invention.
Figure 6 is an exploded view of an exemplary electrode pad according to an embodiment of the present invention.
Figure 7 is an exploded view of an exemplary electrode pad according to an embodiment of the present invention.
Figure 8 illustrates a cross-section of an exemplary method of manufacturing an electrode pad according to an embodiment of the present invention.
Figure 9 illustrates a cross-section of an exemplary method of manufacturing an electrode pad according to an embodiment of the present invention.

### Detailed description

In the following, embodiments of the invention will be discussed in more detail with reference to the appended drawings. It should be understood, however, that the drawings are not intended to limit the invention to the subject-matter depicted in the drawings.

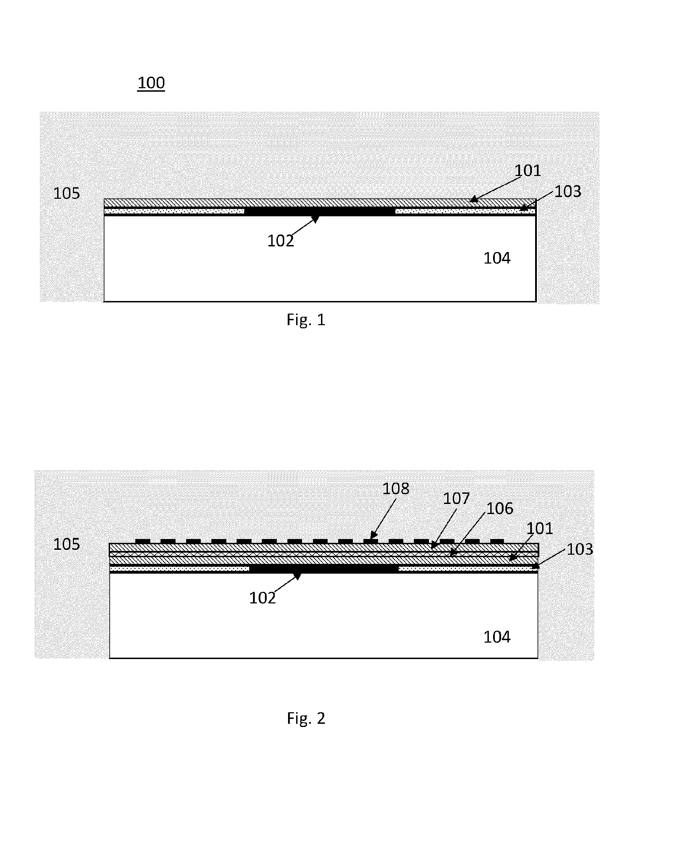

Figure 1 illustrates a cross-section of an exemplary electrode pad 100 for treatment of patients using electro-stimulation. The electrode pad comprises a first non-conductive flexible substrate 101 comprising at least one conductive track 102 disposed on a first side of the non-conductive flexible substrate 101. The non-conductive flexible substrate 101 may made of a flexible printed circuit board (PCB) material, e.g. a polyimide material such as Kapton^{®}, polyethylene terephthalate (PET) or other flexible polymers suitable for electronics. The least one conductive track 102 may preferably be comprise of copper, but any other suitable conductive material may be used.

Silicone, particularly medical grade silicone, is a well-known effective material for use in medical devices that is flexible, conformable and easy to clean, disinfect and re-use. The electrode pad 100 comprises a layer of conductive silicone 104. The layer of conductive silicone 104 is arranged to contact the skin of the patient. The conductive silicone preferably comprises of silicone mixed with conductive materials such as carbon, nickel or silver. Silicone does not readily bond to other materials; it is particularly poor in bonding to metals and plastics without an intermediate primer. A primer 103 is disposed on the first side of the first non-conductive flexible substrate 101 in areas not covered by the at least one conductive track 102. In one embodiment, the primer 103 is a non-conductive primer.

The layer of conductive silicone 104 is disposed over the at least one conductive track 102 and the primer 103. The primer is bonding the first non-conductive flexible substrate 101 to the conductive silicone 104 providing mechanical contact between the at least one conductive track 102 and a first side of the layer of conductive silicone 104. The layer of conductive silicone 104 may preferably be a compression molded layer of conductive silicone. This allows the electrically conductive silicone 104 to be molded and cured with high pressure applied ensured a high pre-load to the conductive track 102 and the conductive silicone 104, whilst bonding the neighboring primed non-conductive flexible substrate 101 to the silicone 104. Although no bond exists between the conductive track 102 and the silicone 104, this ensures reliable electrical connections. It becomes a mechanically locked contact connection.

In one embodiment, the electrode pad 100 further comprising a non-conductive silicone layer 105 covering all sides of the electrode pad other than the second side of the layer of conductive silicone 104. The second side of the layer of conductive silicone 104 is the side of the conductive silicone 104 arranged to contact the skin of the patient.

Figure 2 illustrates a cross-section of an exemplary electrode pad 200 for treatment of patients using electro-stimulation. In addition to the features of the electrode pad 100, the electrode pad 200 further comprises an adhesive 106 disposed on a second side of the first non-conductive flexible substrate 101. The adhesive 106 may be an adhesive film, such as a double-sided adhesive tape. The electrode pad 200 further comprises a second non-conductive flexible substrate 107 comprising at least one conductive track 108 disposed on a first side of the second non-conductive flexible substrate 107, and where a second side of the second non-conductive flexible substrate 107, opposite the first side of the second non-conductive flexible substrate, is arranged on the adhesive 106. The second non-conductive flexible substrate 107 may made of a flexible printed circuit board (PCB) material, e.g. a polyimide material such as Kapton^{®}, polyethylene terephthalate (PET) or other flexible polymers suitable for electronics. The least one conductive track 108 may preferably be comprise of copper, but any other suitable conductive material may be used.

The non-conductive flexible substrate 101, 107 may in some embodiments be a polymer printed circuit board. The polymer printed circuit board may further comprise at least one least one microcontroller in operational connection with circuitry adapted for at least one of measure and regulate heat, measure movement, and receive and display a pad-ID.

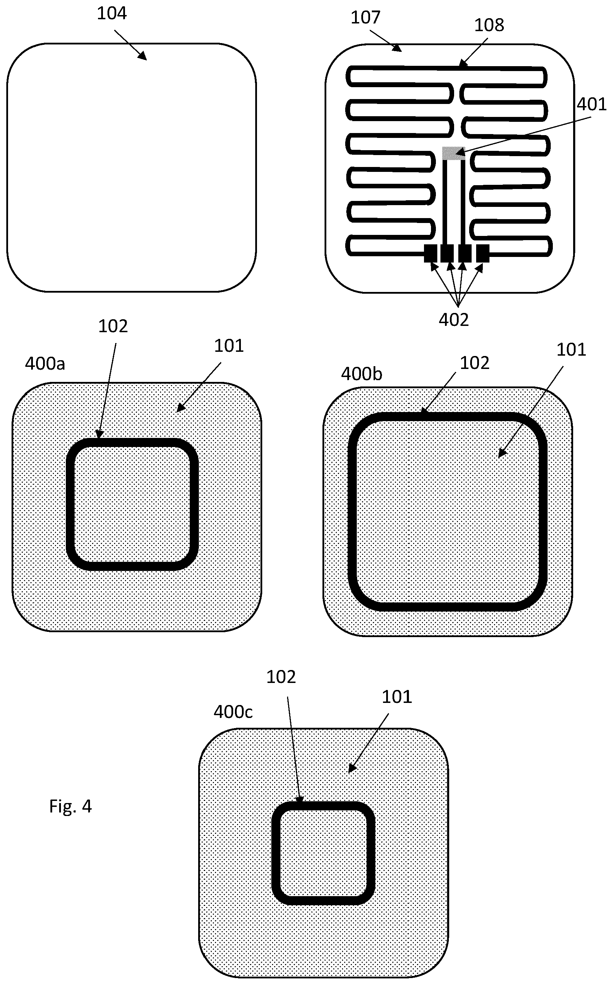

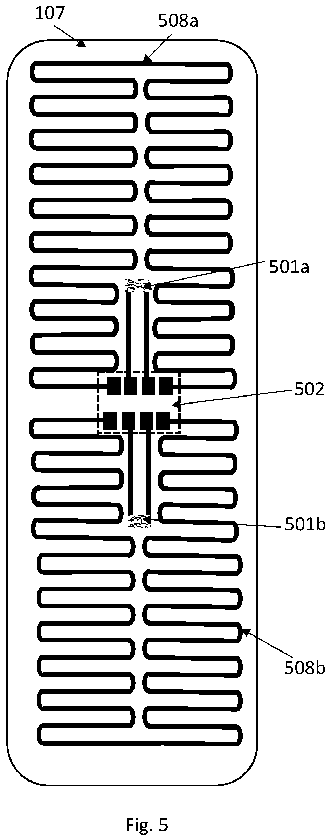

The at least one conductive track 108 on the first side of the second substrate 107 is preferably a heating element. In one embodiment the at least one conductive track 108 is made of copper, 0.1 mm wide and having a thickness of 35µm. The least one conductive track 108 may preferably be arranged in a meandering pattern such as schematically illustrated in Figure 4. Figure 4 further illustrates a heat measuring device 401, such as a thermistor, and connectors 402 to the at least one microcontroller. The electrode pad 200 may as schematically illustrated in Figure 5 comprise at least two conductive tracks 508a, 508b, wherein each of the at least two conductive tracks 508a, 508b is a separate heating element. Figure 5 further illustrates that each heating element is provided with a heating measurement device 501a, 501b, such as a thermistor, and a set of connectors 502 to the at least one microcontroller. The number of separate heating elements depends on the size of the electrode pad. A 50x50mm electrode pad may have one heating elements, whereas a 50x200mm electrode pad may have three heating elements.

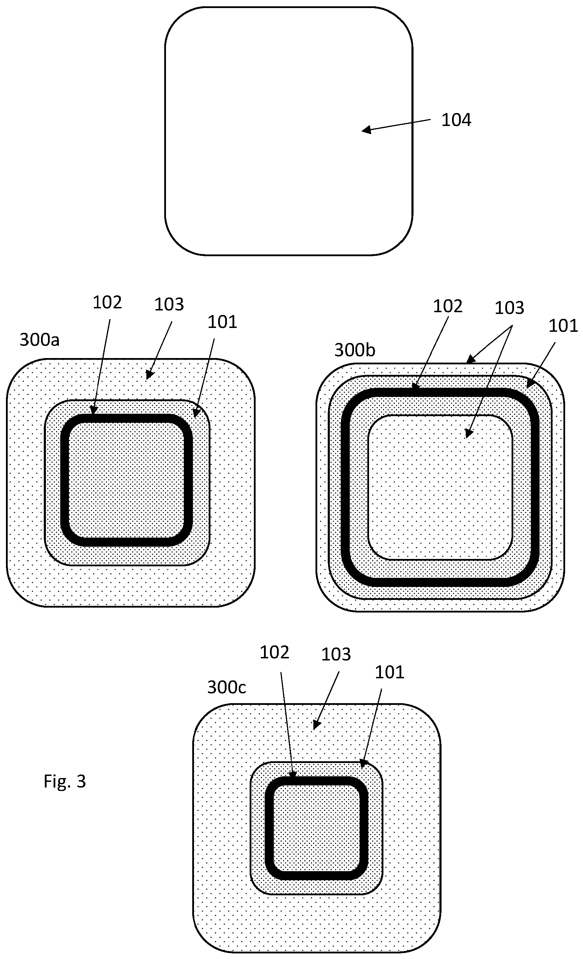

Figure 3 illustrates top views of exemplary electrode pads 300a, 300b, 300c according to three embodiments of the electrode pad 100. Figure 3 shows for size comparison the size of the layer of conductive silicone 104. The conductive track 102 is preferably at an equal distance to the outer perimeter of the layer of conductive silicone 104. The area of the non-conductive flexible substrate 101 of the exemplary electrode pads 300a, 300b, 300c is smaller than the area of the layer of conductive silicone 104. Electrode pads 300a and 300c are configured such that the outer perimeter of the non-conductive flexible substrate 101 is inwards of the outer edge of the perimeter of the conductive silicone 104. Electrode pad 300b is configured with an opening or cutout in the middle of the non-conductive flexible substrate 101. Such openings and reduced outer perimeter enable the following layer of the silicone, the non-conductive silicone 105 to covalently bond to the conductive silicone, thus providing a robust product with reduced dependency on primer-silicone bonding. The conductive track 102 of electrode pad 300a is positioned such that at equidistance is maintained between the tracks and the edge and center of the electrode, to achieve an even current distribution. The conductive track 102 of the electrode pad 300b is positioned nearer the edge of the electrode pad to achieve a higher current density near the edge of the electrode pad 300b, e.g. edge biased. The conductive track 102 of the electrode pad 300c is positioned closer to the center of the electrode to achieve higher current density near the center of the electrode, e.g. center biased.

Figure 4 illustrates top view of exemplary electrode pads 400a, 400b, 400c according to three embodiments of the electrode pad 200. Figure 4 shows for size comparison the size of the layer of the conductive silicone 104. The conductive track 102 is preferably at an equal distance to the outer perimeter of the layer of conductive silicone 104. The area of the non-conductive flexible substrate 101 of the exemplary electrode pads 400a, 400b, 400c is similar to the area of the layer of conductive silicone 104 as well as the area of the second non-conductive flexible substrate 107 to provide electrical and/or thermal insulation to the at least one conductive track 108 on the second non-conductive flexible substrate 107. The conductive track 102 of electrode pad 400a is positioned such that at equidistance is maintained between the tracks and the edge and center of the electrode, to achieve an even current distribution. The conductive track 102 of the electrode pad 400b is positioned nearer the edge of the electrode pad to achieve a higher current density near the edge of the electrode pad 400b, e.g. edge biased. The conductive track 102 of the electrode pad 400c is positioned closer to the center of the electrode to achieve higher current density near the center of the electrode, e.g. center biased.

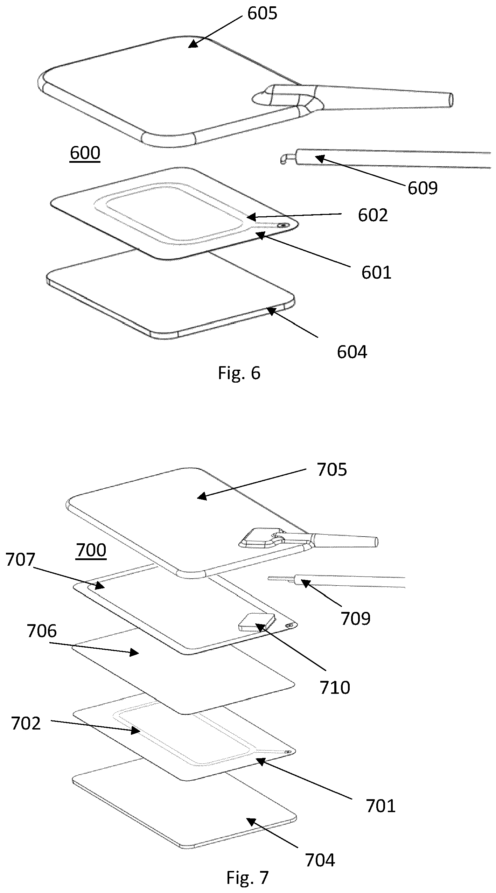

Figure 6 is an exploded view of an exemplary electrode pad 600 according to an embodiment of the electrode pad 100, 300a, 300b, 300c. The electrode pad 600 has a first non-conductive flexible substrate 601. The non-conductive flexible substrate 601 comprises a conductive track 602 and a primer (not shown) not covering the conductive track 602. The conductive track 602 and the primer faces a layer of a layer of conductive silicone 604. The primer is bonding the first non-conductive flexible substrate 601 to the conductive silicone 604 providing mechanical contact between the at least one conductive track 602 and the layer of conductive silicone 604. Figure 6 also shows a cable 609 for electrical connection to the conductive track 602. The electrode pad 600 further comprises a non-conductive silicone layer 605 covering all sides of the electrode pad 600 other than the side of the layer of conductive silicone 604 that is arranged to be positioned against the skin of a patient.

Figure 7 is an exploded view of an exemplary electrode pad 700 according to an embodiment of the electrode pad 200, 400a, 400b, 400c. The electrode pad 700 has a first non-conductive flexible substrate 701. The non-conductive flexible substrate 701 comprises a conductive track 702 and a primer (not shown) not covering the conductive track 702. The conductive track 702 and the primer faces a layer of a layer of conductive silicone 704. The primer is bonding the first non-conductive flexible substrate 701 to the conductive silicone 704 providing mechanical contact between the at least one conductive track 702 and the layer of conductive silicone 704. An adhesive 706 is disposed on the side of the first non-conductive flexible substrate 701 not facing the conductive silicon 704. A second non-conductive flexible substrate 707 comprising a conductive track (not shown) is arranged on the adhesive 706. The second non-conductive substrate 707 comprises at least one microcontroller 710 in operational connection with circuitry adapted for at least one of measure and regulate heat, measure movement, and/or receive and display a pad-ID. Figure 7 also shows a cable 709 for electrical connection to the conductive track 702 and/or the microcontroller 710. The electrode pad 700 further comprises a non-conductive silicone layer 705 covering all sides of the electrode pad 700 other than the side of the layer of conductive silicone 704 that is arranged to be positioned against the skin of a patient.

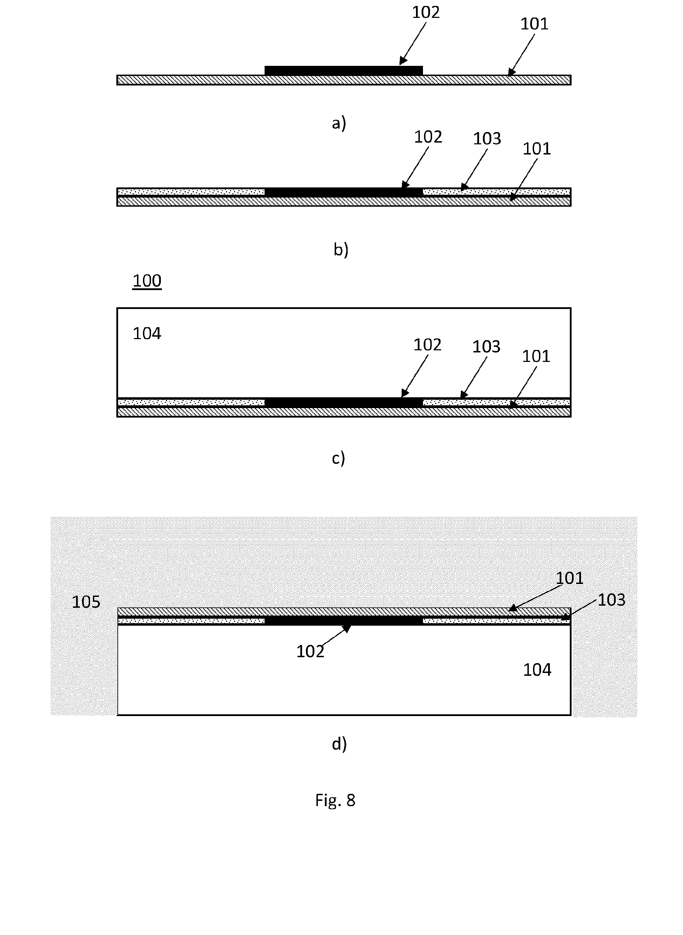

Prior art electrode pads have cables exiting in the middle of the electrode pad. Such cables often conflict with strapping of the electrode to a patient when pad and cable is wrapped around the limb of a patient. This is particularly problematic when many electrode pads are wrapped to a patient. An advantage of the cables 609, 709 illustrated in Figures 7 and 8, exiting the electrode pad 600, 700 at the corner of the electrode pad 600, 700, is that it allows strapping in all directions.

Figure 8 illustrates a cross-section of an exemplary method of manufacturing an electrode pad 100 according to an embodiment of the present invention. The method comprising a first step a) providing the first non-conductive flexible substrate 101 comprising at least one conductive track 102 disposed on a first side of the first non-conductive flexible substrate 101. In the next step b) the at least one conductive track 102 is covered with a mask, and the primer 103 is disposed on the first side of the first non-conductive flexible substrate 101. Then the mask is removed. Then in step c) the layer of conductive silicone 104 is disposed over the at least first conductive track 102 and the primer 103, such that the primer is bonding the first non-conductive flexible substrate 101 to the conductive silicone 104 providing mechanical contact between the at least one conductive track 102 and a first side of the layer of conductive silicone 104.

The method may further comprise step d) of covering all sides of the electrode pad 100 other than the second side of the layer of conductive silicone 104 with a non-conductive silicone layer 105.

The step of disposing a layer of conductive silicone 104 may in one preferable embodiment further comprise the steps of placing the first non-conductive flexible substrate 101 in a molding tool, disposing conductive silicone 104 over the at least a first conductive track 102 and the primer 103 in the molding tool, and applying pressure and heat to the molding tool, such that the layer of conductive silicone 104 is compression molded to the first non-conductive flexible substrate 101. This allows the electrically conductive silicone 104 to be molded and cured with high pressure applied ensuring a high pre-load to the conductive track 102 and the conductive silicone 104, whilst bonding the neighboring primed non-conductive flexible substrate 101 to the silicone 104. Although no bond exists between the conductive track 102 and the silicone 104, this ensures reliable electrical connections. It becomes a mechanically locked contact connection. Alternatively, a liquid silicone rubber injection molding method may be used.

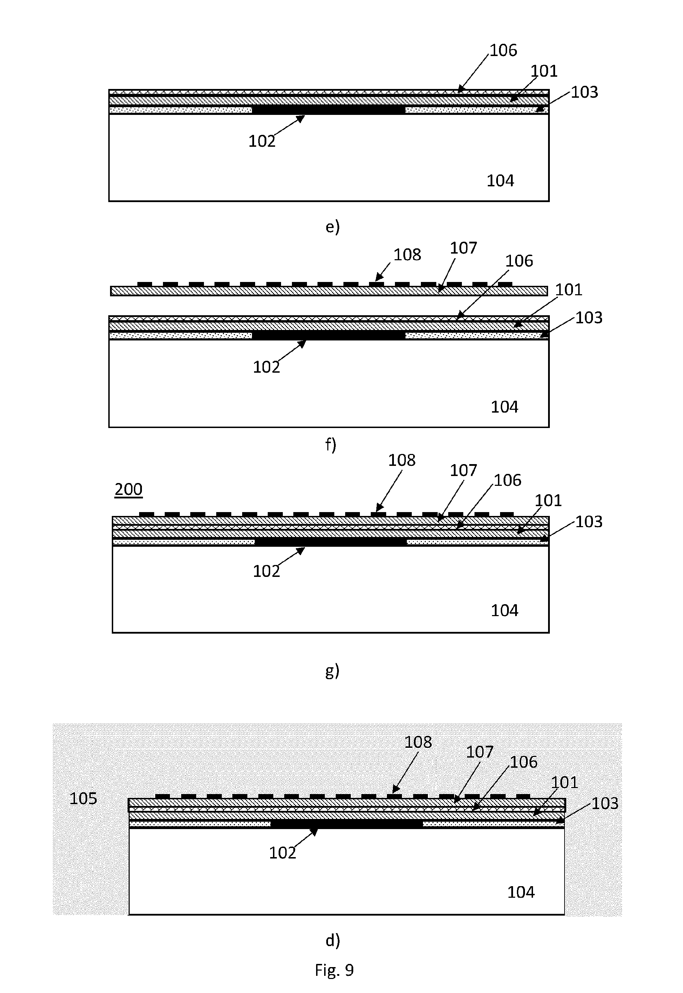

Figure 9 illustrates a cross-section of an exemplary method of manufacturing an electrode pad 200 according to an embodiment of the present invention. The method continues from step c) described above with reference to Figure 5. The method further comprises step e) of disposing an adhesive 106 on the second side of the first non-conductive flexible substrate 101. Then the next step f) comprises providing the second non-conductive flexible substrate 107 comprising the least one conductive track 108 disposed on the first side of the second non-conductive flexible substrate 107. Then the next step g) comprises arranging the second side of the second non-conductive flexible substrate 107, opposite the first side of the second non-conductive flexible substrate 107, on the adhesive 106.

The method may further comprise the step d) of covering all sides of the electrode pad 200 other than the second side of the layer of conductive silicone 104 with a non-conductive silicone layer 105.

In the preceding description, various aspects of the electrode pad and method of manufacture according to the invention have been described with reference to the illustrative embodiment. For purposes of explanation, specific numbers, systems and configurations were set forth in order to provide a thorough understanding of the system and its workings. However, this description is not intended to be construed in a limiting sense. Various modifications and variations of the illustrative embodiment, as well as other embodiments of the method and image processing device, which are apparent to persons skilled in the art to which the disclosed subject matter pertains, are deemed to lie within the scope of the present invention which is defined by the appended claims.

## Claims

1. An electrode pad (100, 200) for electro-stimulation, comprising:
- a first non-conductive flexible substrate (101) comprising at least one conductive track (102) disposed on a first side of the first non-conductive flexible substrate (101),
- a primer (103) disposed on the first side of the first non-conductive flexible substrate (101) in areas not covered by the at least one conductive track (102),
- a layer of conductive silicone (104) disposed over the at least one conductive track (102) and the primer (103), wherein the primer (103) is bonding the first non-conductive flexible substrate (101) to the layer of conductive silicone (104) providing mechanical contact between the at least one conductive track (102) and a first side of the layer of conductive silicone (104).

2. The electrode pad (200) of claim 1, further comprising
- an adhesive (106) disposed on a second side of the first non-conductive flexible substrate (101),
- a second non-conductive flexible substrate (107) comprising at least one conductive track (108) disposed on a first side of the second non-conductive flexible substrate (107), and where a second side of the second non-conductive flexible substrate (107), opposite the first side of the second non-conductive flexible substrate, is arranged on the adhesive (106).

3. The electrode pad (100, 200) of claim 1 or 2, further comprising a non-conductive silicone layer (105) covering all sides of the electrode pad other than the second side of the layer of conductive silicone (104).

4. The electrode pad (100, 200) of any of the preceding claims, wherein the primer (103) is a non-conductive primer.

5. The electrode pad (100, 200) of any of the preceding claims, wherein the layer of conductive silicone (104) is a compression molded layer of conductive silicone.

6. The electrode pad (100, 200) of any of the preceding claims, wherein the non-conductive flexible substrate (101, 107) is a polymer printed circuit board.

7. The electrode pad (100, 200) of claim 6, wherein the polymer printed circuit board comprises at least one microcontroller in operational connection with circuitry adapted for at least one of measure and regulate heat, measure movement, and receive and display a pad-ID.

8. The electrode pad (200) of claim 2, wherein the at least one conductive track (108) on the first side of the second non-conductive flexible substrate (107) is a heating element.

9. The electrode pad (200) of claim 8, wherein the electrode pad (200) comprises at least two conductive tracks (508a, 508b), wherein each of the conductive tracks (508a, 508b) is a separate heating element.

10. The electrode pad (200) of claim 8 or 9, wherein the heating element comprises the least one conductive track (108, 508a, 508b) arranged in a meandering pattern.

11. The electrode pad (100, 200) of any of the preceding claims, wherein the adhesive (106) is an adhesive film.

12. Method of manufacturing an electrode pad for electro-stimulation, the method comprising:
- providing a first non-conductive flexible substrate (101) comprising at least one conductive track (102) disposed on a first side of the first non-conductive flexible substrate (101),
- covering the at least one conductive track (102) with a mask,
- disposing a primer (103) on the first side of the first non-conductive flexible substrate (101),
- removing the mask,
- disposing a layer of conductive silicone (104) over the at least first conductive track (102) and the primer (103), such that the primer is bonding the first non-conductive flexible substrate (101) to the conductive silicone (104) providing mechanical contact between the at least one conductive track (102) and a first side of the layer of conductive silicone (104).

13. The method of claim 12, wherein the method further comprising:
- disposing an adhesive (106) on a second side of the first non-conductive flexible substrate (101),
- providing a second non-conductive flexible substrate (107) comprising at least one conductive track (108) disposed on a first side of the second non-conductive flexible substrate (107),
- arranging a second side of the second non-conductive flexible substrate (107), opposite the first side of the second non-conductive flexible substrate (107), on the adhesive (106).

14. The method of claim 12 or 13, wherein the method further comprising covering all sides of the electrode pad (100, 200) other than the second side of the layer of conductive silicone (104) with a non-conductive silicone layer (105).

15. The method of claim 12, wherein the step of disposing a layer of conductive silicone (104) further comprising:
- placing the first non-conductive flexible substrate (101) in a molding tool,
- disposing conductive silicone (104) over the at least first conductive track (102) and the primer (103) in the molding tool, and
- applying pressure and heat to the molding tool, such that the layer of conductive silicone (104) is compression molded to the first non-conductive flexible substrate (101).

## Patentansprüche

1. Elektrodenpad (100, 200) zur Elektrostimulation, umfassend:
- ein erstes nichtleitendes flexibles Substrat (101), das mindestens eine Leiterbahn (102) umfasst, die auf einer ersten Seite des ersten nichtleitenden flexiblen Substrats (101) angeordnet ist,
- eine Grundierung (103), die auf der ersten Seite des ersten nichtleitenden flexiblen Substrats (101) in Bereichen angeordnet ist, die nicht von der mindestens einen Leiterbahn (102) bedeckt sind,
- eine Schicht aus leitendem Silikon (104), die über der mindestens einen Leiterbahn (102) und der Grundierung (103) angeordnet ist, wobei die Grundierung (103) das erste nichtleitende flexible Substrat (101) mit der Schicht aus leitendem Silikon (104) verbindet, wodurch ein mechanischer Kontakt zwischen der mindestens einen Leiterbahn (102) und einer ersten Seite der Schicht aus leitendem Silikon (104) bereitgestellt wird.

2. Elektrodenpad (200) nach Anspruch 1, ferner umfassend
- einen Klebstoff (106), der auf einer zweiten Seite des ersten nichtleitenden flexiblen Substrats (101) angeordnet ist,
- ein zweites nichtleitendes flexibles Substrat (107), das mindestens eine Leiterbahn (108) umfasst, die auf einer ersten Seite des zweiten nichtleitenden flexiblen Substrats (107) angeordnet ist, und wobei eine zweite Seite des zweiten nichtleitenden flexiblen Substrats (107) gegenüber der ersten Seite des zweiten nichtleitenden flexiblen Substrats auf dem Klebstoff (106) vorgesehen ist.

3. Elektrodenpad (100, 200) nach Anspruch 1 oder 2, ferner umfassend eine nichtleitende Silikonschicht (105), die alle Seiten des Elektrodenpads mit Ausnahme der zweiten Seite der Schicht aus leitendem Silikon (104) bedeckt.

4. Elektrodenpad (100, 200) nach einem der vorhergehenden Ansprüche, wobei die Grundierung (103) eine nichtleitende Grundierung ist.

5. Elektrodenpad (100, 200) nach einem der vorhergehenden Ansprüche, wobei die Schicht aus leitendem Silikon (104) eine pressgeformte Schicht aus leitendem Silikon ist.

6. Elektrodenpad (100, 200) nach einem der vorhergehenden Ansprüche, wobei das nichtleitende flexible Substrat (101, 107) eine Polymerleiterplatte ist.

7. Elektrodenpad (100, 200) nach Anspruch 6, wobei die Polymerleiterplatte mindestens einen Mikrocontroller in Betriebsverbindung mit einer Schaltung umfasst, die für mindestens eines von Messen und Regulieren von Wärme, Messen von Bewegung und Empfangen und Anzeigen einer Pad-ID ausgelegt ist.

8. Elektrodenpad (200) nach Anspruch 2, wobei die mindestens eine Leiterbahn (108) auf der ersten Seite des zweiten nichtleitenden flexiblen Substrats (107) ein Heizelement ist.

9. Elektrodenpad (200) nach Anspruch 8, wobei das Elektrodenpad (200) mindestens zwei Leiterbahnen (508a, 508b) umfasst, wobei jede der Leiterbahnen (508a, 508b) ein separates Heizelement ist.

10. Elektrodenpad (200) nach Anspruch 8 oder 9, wobei das Heizelement die mindestens eine Leiterbahn (108, 508a, 508b) umfasst, die in einem mäanderförmigen Muster vorgesehen ist.

11. Elektrodenpad (100, 200) nach einem der vorhergehenden Ansprüche, wobei der Klebstoff (106) ein Klebstofffilm ist.

12. Verfahren zur Herstellung eines Elektrodenpads zur Elektrostimulation, wobei das Verfahren Folgendes umfasst:
- Bereitstellen eines ersten nichtleitenden flexiblen Substrats (101), das mindestens eine Leiterbahn (102) umfasst, die auf einer ersten Seite des ersten nichtleitenden flexiblen Substrats (101) angeordnet ist,
- Bedecken der mindestens einen Leiterbahn (102) mit einer Maske,
- Anordnen einer Grundierung (103) auf der ersten Seite des ersten nichtleitenden flexiblen Substrats (101),
- Entfernen der Maske,
- Anordnen einer Schicht aus leitendem Silikon (104) über der mindestens ersten Leiterbahn (102) und der Grundierung (103), sodass die Grundierung das erste nichtleitende flexible Substrat (101) mit dem leitenden Silikon (104) verbindet, wodurch ein mechanischer Kontakt zwischen der mindestens einen Leiterbahn (102) und einer ersten Seite der Schicht aus leitendem Silikon (104) bereitgestellt wird.

13. Verfahren nach Anspruch 12, wobei das Verfahren ferner Folgendes umfasst:
- Anordnen eines Klebstoffs (106) auf einer zweiten Seite des ersten nichtleitenden flexiblen Substrats (101),
- Bereitstellen eines zweiten nichtleitenden flexiblen Substrats (107), das mindestens eine Leiterbahn (108) umfasst, die auf einer ersten Seite des zweiten nichtleitenden flexiblen Substrats (107) angeordnet ist,
- Vorsehen einer zweiten Seite des zweiten nichtleitenden flexiblen Substrats (107) gegenüber der ersten Seite des zweiten nichtleitenden flexiblen Substrats (107) auf dem Klebstoff (106).

14. Verfahren nach Anspruch 12 oder 13, wobei das Verfahren ferner Bedecken aller Seiten des Elektrodenpads (100, 200) mit Ausnahme der zweiten Seite der Schicht aus leitendem Silikon (104) mit einer nichtleitenden Silikonschicht (105) umfasst.

15. Verfahren nach Anspruch 12, wobei der Schritt des Anordnens einer Schicht aus leitendem Silikon (104) ferner Folgendes umfasst:
- Platzieren des ersten nichtleitenden flexiblen Substrats (101) in einem Formwerkzeug,
- Anordnen von leitendem Silikon (104) über der mindestens ersten Leiterbahn (102) und der Grundierung (103) in dem Formwerkzeug, und
- Aufbringen von Druck und Wärme auf das Formwerkzeug, sodass die Schicht aus leitendem Silikon (104) an das erste nichtleitende flexible Substrat (101) pressgeformt wird.

## Revendications

1. Plot d'électrode (100, 200) pour électro-stimulation, comprenant :
- un premier substrat flexible non conducteur (101) comprenant au moins une piste conductrice (102) disposée sur un premier côté du premier substrat flexible non conducteur (101),
- un apprêt (103) disposé sur le premier côté du premier substrat flexible non conducteur (101) dans des zones non couvertes par l'au moins une piste conductrice (102),
- une couche de silicone conducteur (104) disposée sur l'au moins une piste conductrice (102) et l'apprêt (103), dans lequel l'apprêt (103) lie le premier substrat flexible non conducteur (101) à la couche de silicone conducteur (104) fournissant un contact mécanique entre l'au moins une piste conductrice (102) et un premier côté de la couche de silicone conducteur (104).

2. Plot d'électrode (200) selon la revendication 1, comprenant en outre :
- un adhésif (106) disposé sur un second côté du premier substrat flexible non conducteur (101),
- un second substrat flexible non conducteur (107) comprenant au moins une piste conductrice (108) disposée sur un premier côté du second substrat flexible non conducteur (107), et où un second côté du second substrat flexible non conducteur (107), opposé au premier côté du second substrat flexible non conducteur, est disposé sur l'adhésif (106).

3. Plot d'électrode (100, 200) selon la revendication 1 ou 2, comprenant en outre une couche de silicone non conducteur (105) recouvrant tous les côtés du plot d'électrode autres que le second côté de la couche de silicone conducteur (104).

4. Plot d'électrode (100, 200) selon l'une quelconque des revendications précédentes, dans lequel l'apprêt (103) est un apprêt non conducteur.

5. Plot d'électrode (100, 200) selon l'une quelconque des revendications précédentes, dans lequel la couche de silicone conducteur (104) est une couche moulée par compression de silicone conducteur.

6. Plot d'électrode (100, 200) selon l'une quelconque des revendications précédentes, dans lequel le substrat flexible non conducteur (101, 107) est une carte de circuit imprimé polymère.

7. Plot d'électrode (100, 200) selon la revendication 6, dans lequel la carte de circuit imprimé polymère comprend au moins un microcontrôleur en connexion opérationnelle avec un circuit adapté pour effectuer au moins l'un de la mesure et la régulation de la chaleur, la mesure du mouvement, et la réception et l'affichage d'un ID de plot.

8. Plot d'électrode (200) selon la revendication 2, dans lequel l'au moins une piste conductrice (108) sur le premier côté du second substrat flexible non conducteur (107) est un élément chauffant.

9. Plot d'électrode (200) selon la revendication 8, dans lequel le plot d'électrode (200) comprend au moins deux pistes conductrices (508a, 508b), dans lequel chacune des pistes conductrices (508a, 508b) est un élément chauffant séparé.

10. Plot d'électrode (200) selon la revendication 8 ou 9, dans lequel l'élément chauffant comprend l'au moins une piste conductrice (108, 508a, 508b) agencée selon un motif sinueux.

11. Plot d'électrode (100, 200) selon l'une quelconque des revendications précédentes, dans lequel l'adhésif (106) est un film adhésif.

12. Procédé de fabrication d'un plot d'électrode pour électro-stimulation, le procédé comprenant :
- la fourniture d'un premier substrat flexible non conducteur (101) comprenant au moins une piste conductrice (102) disposée sur un premier côté du premier substrat flexible non conducteur (101),
- la couverture de l'au moins une piste conductrice (102) avec un masque,
- la disposition d'un apprêt (103) sur le premier côté du premier substrat flexible non conducteur (101),
- le retrait du masque,
- la disposition d'une couche de silicone conducteur (104) sur l'au moins une première piste conductrice (102) et l'apprêt (103), de sorte que l'apprêt lie le premier substrat flexible non conducteur (101) au silicone conducteur (104) fournissant un contact mécanique entre l'au moins une piste conductrice (102) et un premier côté de la couche de silicone conducteur (104).

13. Procédé selon la revendication 12, dans lequel le procédé comprend en outre :
- la disposition d'un adhésif (106) sur un second côté du premier substrat flexible non conducteur (101),
- la fourniture d'un second substrat flexible non conducteur (107) comprenant au moins une piste conductrice (108) disposée sur un premier côté du second substrat flexible non conducteur (107),
- la disposition d'un second côté du second substrat flexible non conducteur (107), opposé au premier côté du second substrat flexible non conducteur (107), sur l'adhésif (106).

14. Procédé selon la revendication 12 ou 13, dans lequel le procédé comprend en outre la couverture de tous les côtés du plot d'électrode (100, 200) autres que le second côté de la couche de silicone conducteur (104) avec une couche de silicone non conducteur (105).

15. Procédé selon la revendication 12, dans lequel l'étape de disposition d'une couche de silicone conducteur (104) comprend en outre :
- le placement du premier substrat flexible non conducteur (101) dans un outil de moulage,
- la disposition du silicone conducteur (104) sur l'au moins une première piste conductrice (102) et l'apprêt (103) dans l'outil de moulage, et
- l'application de pression et de chaleur à l'outil de moulage, de sorte que la couche de silicone conducteur (104) soit moulée par compression sur le premier substrat flexible non conducteur (101).
